# EUROPEAN PATENT SPECIFICATION

(11) **EP 4 085 126 B1**
(45) Date of publication and mention of the grant of the patent: **03.07.2024**
(21) Application number: 20842243.6
(22) Date of filing: 30.12.2020
(51) Int. Cl.: C12M 1/00, C12M 1/12, C12M 1/34

(54) **SINGLE-USE CELL CULTURE CONTAINER WITH TWO OR MORE IN-SITU ONLINE SENSORS**
EINWEGZELLZÜCHTUNGSBEHÄLTER MIT ZWEI ODER MEHREREN IN-SITU-ONLINE-SENSOREN
RÉCIPIENT DE CULTURE CELLULAIRE À USAGE UNIQUE AVEC DEUX OU PLUSIEURS CAPTEURS IN SITU EN LIGNE

(30) Priority: 02.01.2020 EP 20150132
(43) Date of publication of application: 09.11.2022
(73) Proprietor: F. Hoffmann-La Roche AG, 4070 Basel (CH)
(72) Inventor: SCHWALD, Christian, 82377 Penzberg (DE)
(74) Representative: Skolaut, Alexander
(86) International application number: PCT/EP2020/088013
(87) International publication number: WO 2021/136798

(56) References cited:
- US-A1- 2019 048 305
- US-A1- 2019 153 381
- US-B1- 8 026 096

## Description

The present invention is in the field of mammalian cell cultivation. In more detail, the current invention inter alia relates to a single-use small volume cell culture container, also termed single-use small volume bioreactor (SUSVB), comprising one or more, especially two or more, in-situ sensors, as well as its use in small volume cultivations of mammalian cells.

### Background

The acceleration of bioprocess development for biologics and vaccines can be enabled by automated high throughput technologies, which can alleviate the significant resource burden from the multi-factorial statistical experimentation required for controlling product quality attributes of complex biologics (Bareither, R., et al., Biotechnol. Bioeng. 110 (2013) 3126-3138).

Recently, Bareither, R., et al., provided the proof of concept evaluations of an automated disposable small scale reactor for high throughput upstream process development by establishing a small scale stirred tank disposable 250 mL reactor as similar to those of lab and pilot scale with respect to process performance for industrial biologics processes for therapeutic protein and monoclonal antibody production using CHO cell culture, Pichia pastoris and E. coli (Biotechnol. Bioeng. 110 (2013) 3126-3138). This included similar growth, cell viability, product titer, and product quality. The technology was shown to be robust across multiple runs and met the requirements for the ability to run high cell density processes (>400 g/L wet cell weight) with exponential feeds and sophisticated event triggered processes.

Single use cell culture containers are well known in the art, such as, e.g., the ambr^{®} 15 and 250 systems marketed by Sartorius Stedim Biotech.

US 2019/048305 reported a perfusion bioreactor and method for using same to perform a continuous cell culture, wherein the reactor comprises a single sensor port (240) with a single sensor (254) mounted thereto.

US 2019/153381 reported a perfusion bioreactor and related methods of use, wherein the reactor comprises as one sensing element a RAMAN sensor in the headspace, i.e. a cultivation-medium contactless sensor.

US 8,026,096 reported in vivo active erythropoietin produced in insect cells using bioreactors of two liters or more working volume.

### Summary of the Invention

Commercially available single-use small volume bioreactors (SUSVBs) provide at best for the control of temperature, dissolved oxygen and pH and only have capabilities for feed addition(s) and sampling. However, there is no option for a second in-situ, i.e. in direct contact with the cultivation medium, metabolite sensor. Thus, if, e.g., beside the pH control an additional monitoring of glucose is required, inevitably off-line analysis by sampling has to be done. This increases amongst other things the handling efforts as well as the risk of contamination of the cultivation. It has now been found by the current inventors that by using a small volume bioreactor according to the current invention timelines for analytics and for developing biological processes can be shortened. Additionally costs can be reduced. All that is achieved without losing the transferability with and to later large-scale processes.

One aspect of the current invention is a small volume bioreactor, as defined in claim 1, comprising one or more in-situ sensors, wherein at least one in-situ sensor is for the determination of glucose.

In one preferred embodiment, the small volume bioreactor comprises two or more in-situ sensors, wherein one in-situ sensor is for the determination of glucose and one in-situ sensor is for the determination of the pH value. In one preferred embodiment, both sensors are submersed sensors, i.e. in direct contact with the cultivation medium.

The pH sensor is a pH electrode.

In one embodiment, the glucose sensor is an electrochemical and/or enzyme-based sensor.

In the inventive embodiment the small volume bioreactor comprises two or more in-situ sensors, wherein one in-situ sensor is for the determination of glucose and one in-situ sensor is for the determination of the pH value, wherein the pH sensor is a pH electrode and the glucose sensor is an electrochemical and/or enzyme-based sensor. In one preferred embodiment, both sensors are submersed sensors, i.e. in direct contact with the cultivation medium.

In one embodiment, the small volume bioreactor has a working volume of from 20 ml to 350 ml. In one embodiment, the working volume is from 25 ml to 300 ml. In one embodiment, the working volume is from 50 ml to 280 ml. In one embodiment, the working volume is from 55 ml to 270 ml.

In one embodiment, the small volume bioreactor is operated at a volume of from 60 ml to 260 ml. In one embodiment, the small volume bioreactor is operated at a volume of from 90 ml to 250 ml.

In one embodiment, the small volume bioreactor has a total volume of 500 ml or less. In one embodiment, the small volume bioreactor has a total volume of 450 ml or less. In one embodiment, the small volume bioreactor has a total volume of 400 ml or less.

In one embodiment, the small volume bioreactor comprises a cultivation vessel (105) and a reactor head plate (104).

In one embodiment, the at least two in-situ sensors further comprise a lactate sensor.

In one embodiment, the supply port area comprises one to four inlets for liquids connected to individual feed lines (118, 119, 120, 121) and the sparger gas inlet (116).

In one embodiment two impeller (109, 112) are connected to the stirrer shaft

In one embodiment, the small volume bioreactor comprises the sparger gas inlet (116) connected to a headspace gas inlet (132).

In one embodiment, the glucose sensor determines the glucose concentration every 20 seconds and/or the glucose sensor provides a signal if there is a change in glucose concentration. In one embodiment, the determined glucose concentration value is transmitted by wire or wireless to the computer. In one embodiment, the transmittal of the glucose concentration is by WI-FI, RFID or Bluetooth. In one embodiment, the glucose sensor has a working range of up to 8 g/l glucose or up to 3 g/l glucose. In one embodiment, the glucose sensor is an electrochemical and/or enzyme-based sensor. In one embodiment, the glucose sensor is a screen-printed electrode coated with an immobilized enzyme. In one embodiment, the glucose sensor base material is polymer USP class VI.

In one embodiment, the small volume bioreactor is a single-use bioreactor.

In one embodiment, the cultivation vessel (105) and the reactor head plate (104) are made of non-metal material. In one embodiment the cultivation vessel (105) and the reactor head plate (104) are (made of) plastic.

In one embodiment, the reactor head plate (104) further comprises a sampling port (102).

In one embodiment, the small volume bioreactor is sterilizable.

In one embodiment, the small volume bioreactor is a radiation sterilized small volume bioreactor. In one embodiment the small volume bioreactor is a twice radiation sterilized small volume bioreactor. In one embodiment, the radiation is beta and/or gamma radiation.

### Detailed Descrintion of the Invention

In contrast to the early years of biotherapeutic production it has been well accepted in recent years that for the development of therapeutic proteins, such as antibodies, the decisive parameter is not only product titer but also specific product quality attributes such as, without being limiting, the by-product profile or the glycosylation profile/pattern (see, e.g., Bareither, R. and Pollard, D., Biotech. Prog. 155 (2011) 217-224).

Thus, there is the need to assess and engineer these properties as early as possible, best already during clone selection and process development. As this is, in addition, no none-time process, consecutive rounds of experimentation using multiple parallel reactors are typically required. Thus, the method needs to be high-throughput suitable.

In view of ecological and economical constrains no at-scale cultivations can be used for process development. Thus, a suitable scale-down system that is proven to reliably mirror the later commercial (large) scale process and process performance is employed.

The invention as claimed in claim 1 is defined by a small volume bioreactor comprising
a cultivation vessel (105) that
   - has a working volume of from 20 ml to 350 ml,
   - comprises a stirrer shaft (108) with at least one impeller (112) affixed thereto,
   - comprises a feed pipe (107) comprising i) a sparging tube connected to a sparger (127) at its end, and ii) at least one feed line (118) with an opening at its end,
   - comprises two or more baffles (114; 126) extending from the wall of the cultivation vessel (105) in direction to the center of the cultivation vessel (105) (perpendicular to the wall of the cultivation vessel), and
   - a reactor head plate (104),
wherein the reactor head plate (104) comprises
   - a fitting (122) for connecting the drive axis of a motor to the stirrer shaft (108),
   - a sparger gas inlet (116) connected to the sparging tube in the feed pipe (107), optionally a gas inlet connected to the headspace (132),
   - a gas outlet connected to the headspace of the cultivation vessel (117),
   - at least one inlet for liquids of a feed line (118) being part of the feed pipe (107),
   - one in-situ sensor port (130) with a pH electrode (101) mounted thereto, and
   - a supply port area (133) comprising the sparger gas inlet and the inlet of the at least one feed line (118),
wherein the cultivation vessel (105) and the reactor head plate (104) are both substantially made of non-metal material,
characterized in that the small volume bioreactor comprises an in-situ glucose sensor.

The further embodiments are defined in the dependent claims 2-8 and a method is defined in claim 9 for cultivating a mammalian cell using the small bioreactor as defined in claim 1.

### GENERAL DEFINITIONS

The term "antibody" denotes a protein consisting of one or more polypeptide(s) substantially encoded by immunoglobulin genes. The recognized immunoglobulin genes include the different constant region genes as well as the myriad immunoglobulin variable region genes. Antibodies can exist in a variety of formats, including, for example, Fv, Fab, and F(ab)₂ as well as single chains (scFv) or diabodies or triabodies, as monovalent, divalent, trivalent, tetravalent, and pentavalent and hexavalent forms, as well as monospecific, bispecific, trispecific or tetraspecific antibodies.

A "polypeptide" is a polymer consisting of amino acids joined by peptide bonds, whether produced naturally or synthetically. Polypeptides of less than about 20 amino acid residues may be referred to as "peptides", whereas molecules consisting of two or more polypeptides or comprising one polypeptide of more than 100 amino acid residues may be referred to as "proteins". A polypeptide may also comprise non-amino acid components, such as carbohydrate groups, metal ions, or carboxylic acid esters. The non-amino acid components may be added by the cell, in which the polypeptide is produced, and may vary with the type of cell. Polypeptides are defined herein in terms of their amino acid backbone structure or the nucleic acid encoding the same. Additions such as carbohydrate groups are generally not specified, but may be present nonetheless.

The term "in-situ" sensors denotes a sensor that has direct physical contact with a cultivation medium.

### EMBODIMENTS OF THE SMALL VOLUME BIOREACTOR ACCORDING TO THE CURRENT INVENTION

Commercially available single-use small volume bioreactors (SUSVBs) provide means for the control of at most temperature, dissolved oxygen and pH, whereof only the pH value is controlled by an in-situ sensor. Additionally, these SUSVBs have ports for the addition of nutrients or correction fluids as well as capabilities for sampling. Nevertheless, a second in-situ metabolite sensor is not available due to the limited diameter/size and area of the head plate (104). Thus, if, e.g., besides an in-situ pH electrode an additional monitoring of glucose is required this can only be done by off-line analysis using sampling.

It has now been found by the current inventors that it is possible by mounting an additional glucose sensor to the same area as that used for the liquid and gas supply lines, i.e. the supply port area (133), a sterilizable SUSVB can be provided that comprises two in-situ sensors.

The provision of a glucose sensor in an SUSVB according to the current invention allows for continuous in-situ determination of glucose concentration. Thereby the previously required sampling, i.e. the taking of samples, for the determination of glucose is alleviated. The presence of a glucose sensor in the SUSVB allows for the determination of glucose concentration in real-time, i.e. with no time offset. Thereby the cultivation's growth behavior and metabolic state can be monitored more closely, i.e. corrective measures can be taken earlier than with sampling.

It is especially advantageous that with the SUSVB according to the current invention the determination of glucose can be made without interfering with the cultivation, which is required by sampling.

Thus, by integrating an in-situ glucose sensor directly into the SUSVB one or more of the following drawbacks can be circumvented: lower cell density; lower product yield; environmental changes, such as carbon dioxide, temperature, pH agitation; metabolic stress; changed gene expression; changes in cultivation volume; and most importantly contamination.

In one embodiment, the glucose sensor determines the glucose concentration every 20 seconds and/or the glucose sensor provides a signal if there is a change in glucose concentration. In one embodiment, the determined glucose concentration value is transmitted by wire or wireless to the computer. In one embodiment, the transmittal of the glucose concentration is by Wi-Fi, RFID or Bluetooth.

By the presence of a glucose sensor in the SUSVB, it is now possible to control glucose concentration online.

In one embodiment, the glucose sensor has a working range of up to 8 g/l glucose or up to 3 g/l glucose.

In one embodiment, the glucose sensor is an electrochemical and/or enzyme-based sensor. In one embodiment, the glucose sensor is a screen-printed electrode coated with an immobilized enzyme. In one embodiment, the glucose sensor base material is polymer USP class VI.

USP class VI denotes the official US Pharmacopeia (USP) biocompatibility class VI. Therein the requirements for a plastic material to be biocompatible are regulated. Class VI is the class with the most stringent requirements comparable to a pharmaceutical compound marketing registration. This is also regulated in the German Industrial Standard DIN-ISO-10993.

The detection of glucose with an electrochemical and/or enzyme-based sensor is based on the oxidation of glucose by glucose-oxidase. Glucose-oxidase is an enzyme that catalyzes the oxygen-independent oxidation of the C1-residue of glucose. The sensor detects the thereby produced hydrogen peroxide.

In one embodiment, the glucose sensor is gamma-irradiation sterilizable.

In one embodiment, the entire glucose sensor has a total length of 40-500 mm. In one preferred embodiment, the glucose sensor has a total length of 75 to 350 mm. In one embodiment, the electrode or the enzyme-covered area of the glucose sensor has a width of 5-20 mm.

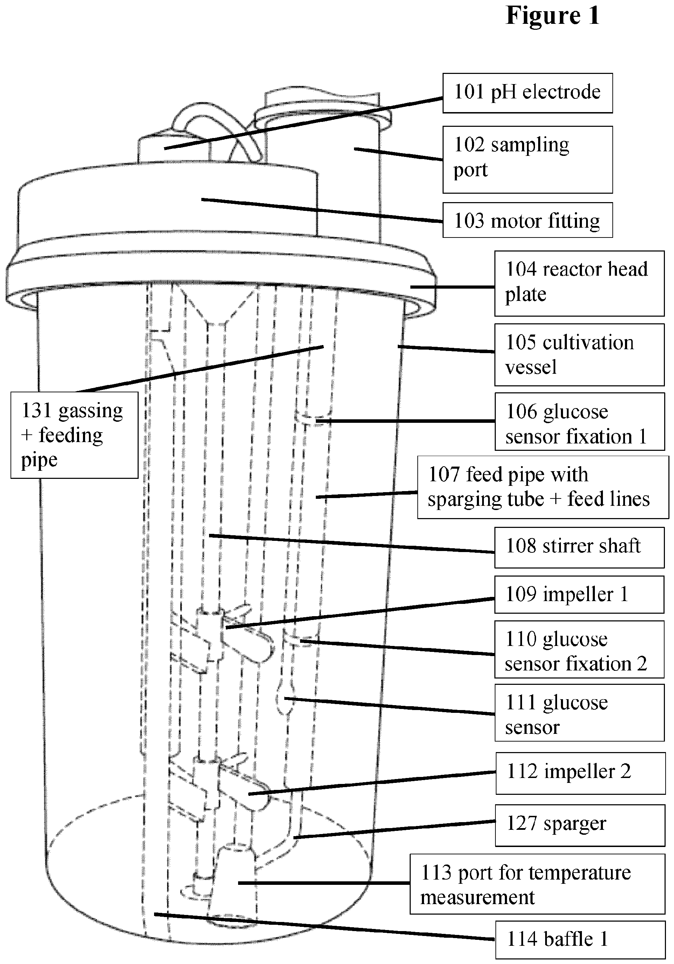

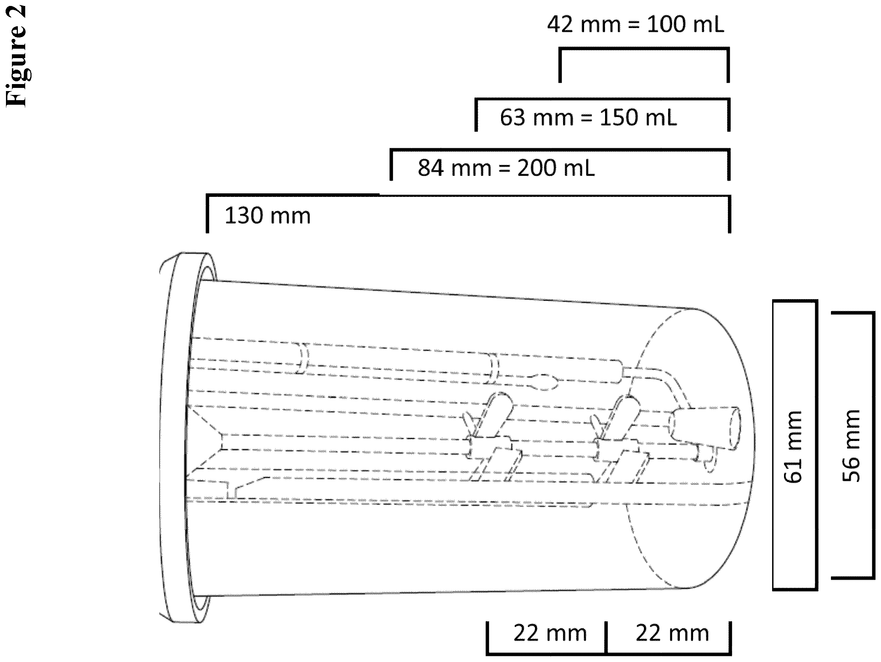

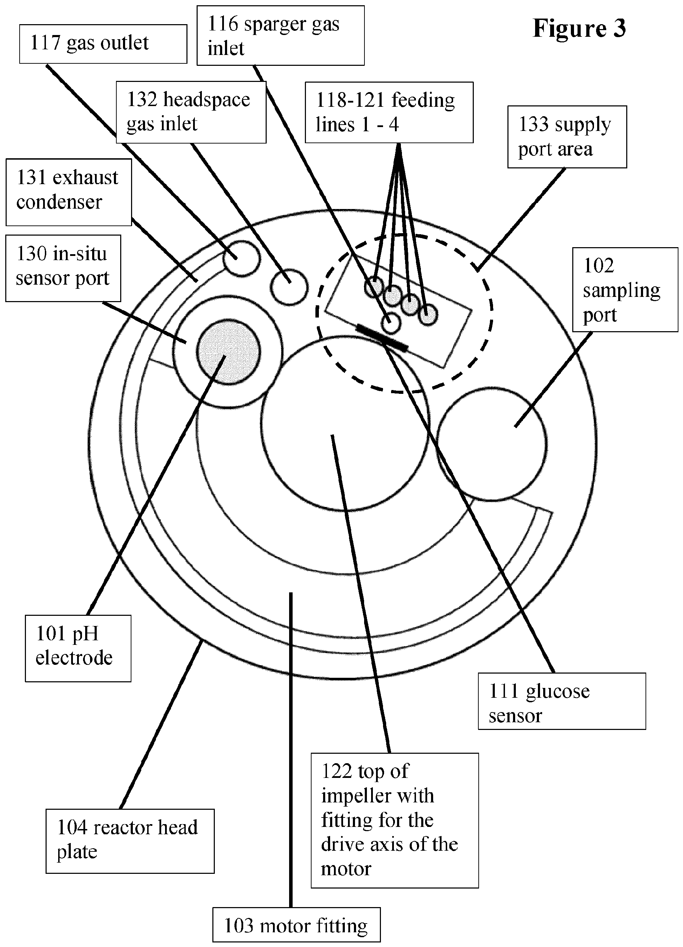

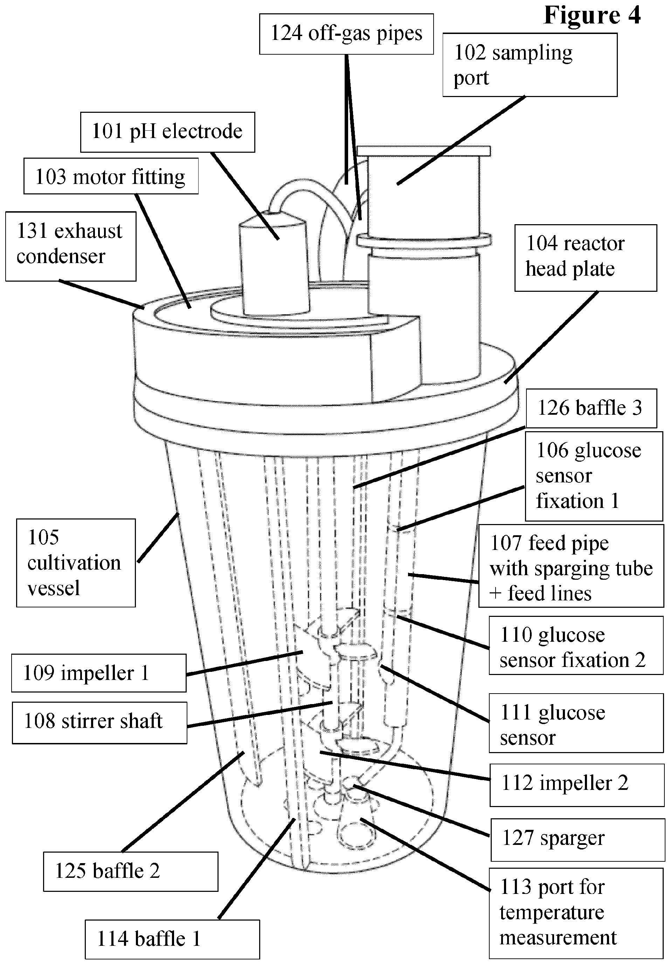

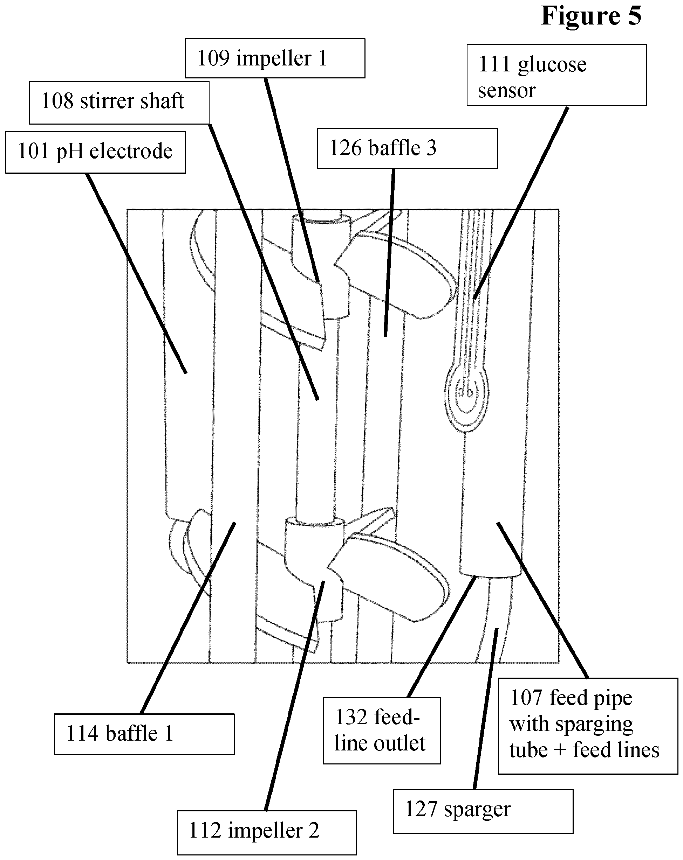

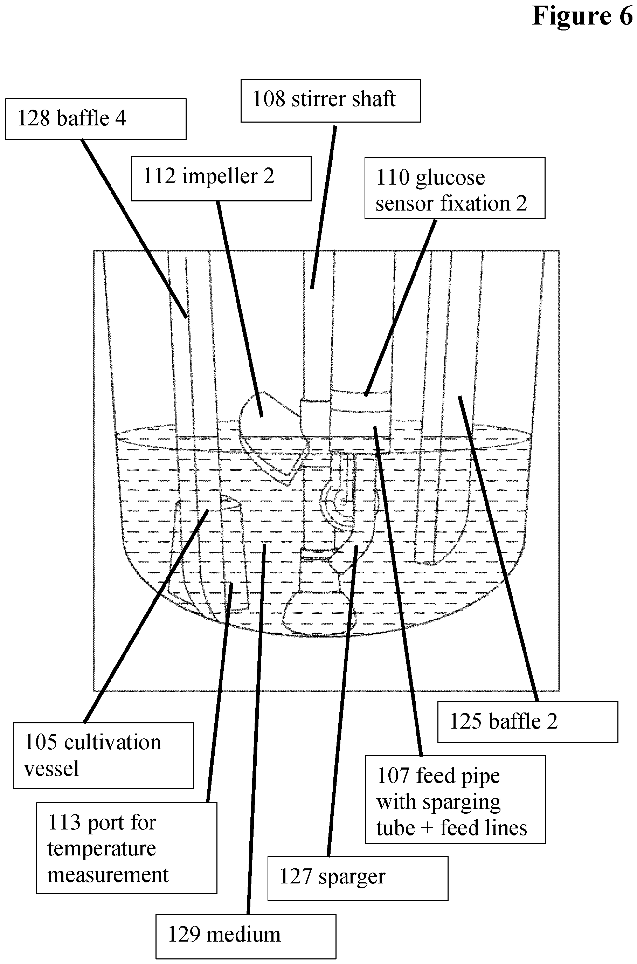

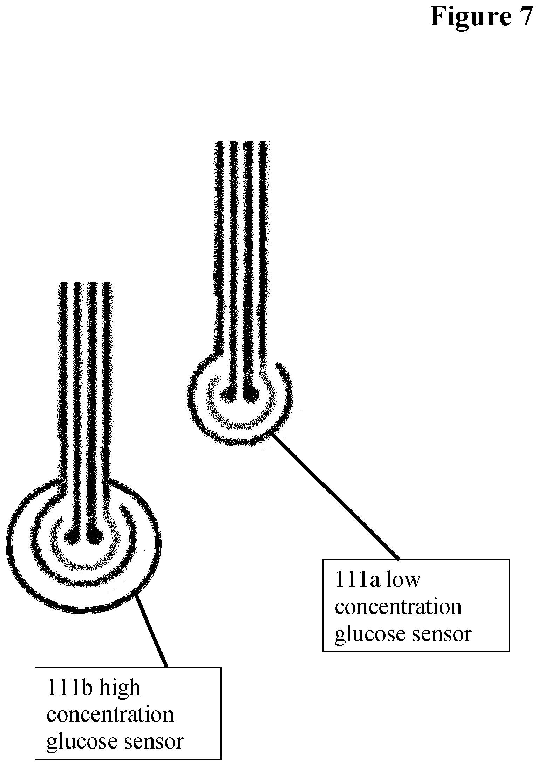

In one preferred embodiment, the SUSVB (see also Figures 1 to 7) has one or more of
- a working volume of from 20 ml to 350 ml, of from 25 ml to 300 ml, of from 50 ml to 280 ml, of from 55 ml to 270 ml, or of from 95 ml to 255 ml;
- a stirrer including a stirrer drive shaft fitted with either two Rushton impellers (about 20 mm diameter, about 30 mm spacing) for microbial culture, or two pitched blade impellers for mammalian cell culture (about 25 mm diameter, about 30 mm spacing);
- two or four equally spaced baffles (about 6.25 mm width) placed on the (vertical) side wall of the reactor and extending in direction of the center of the vessel, i.e. that are perpendicular to the cultivation vessel's inner wall;
- a motor (150 rpm to 3,000 rpm; such as e.g. a brushless electric servo motor) coupled directly to the stirrer drive shaft; in one preferred embodiment an electric d.c. motor (e.g. RE-max 17 series, Maxon, Switzerland);
- a reactor head plate with tubing lines with sterile filters for subsurface additions of gas and liquid and an additional outlet for vent gas;
- a dissolved oxygen sensor fluorescence patch (e.g. from the company PreSens) embedded into the base of the reactor and controlled by cascade of agitation and/or aeration; in one preferred embodiment with a fast response (<2 s) dO2 probe; the measurement interval is between 10 and 15 second, in one preferred embodiment about 12 seconds;
- a gel electrode for pH determination using a three point calibration carried out prior to autoclaving, and a one-point calibration following media addition;
- a liquid filled temperature control jacket; or a temperature control metal block (in one preferred embodiment made of aluminum); the SUSVB can be positioned thereon;
- a control station containing the fluorescence reader for the dissolved oxygen sensor and the temperature probe, individual sensors for vent gas analysis of oxygen (e.g. an electrochemical detector) and carbon dioxide (e.g. an infrared detector); the SUSVB can be positioned thereon;
- an indented section of the base of the vessel wall for measuring the temperature;
- a temperature controlled (clamp) plate (6 °C) adapted to the design of the head plate for vent gas humidity control;
- up to four liquid feeds (e.g. for pH control reagents (acid and base), nutrient) with individual pumps (e.g., syringe pumps) allowing single bolus additions (e.g., with a volume of from 10 µL to 10 mL), continuous feeds (e.g., with different profiles, such as linear or exponential; with flow rates of from 20 nL/h to 20 mL/h); in one preferred embodiment about 150 µL/h;
- a sparging tube (for the delivery of gas (air)); the tube may have an open pipe configuration whereby the gas outlet is directly beneath the bottom impeller;
- a headspace gas inlet for gassing the cultivation medium headspace;
- a multiconfigurable inlet gas manifold (e.g., for blending of air, pure air, oxygen, nitrogen, carbon dioxide) comprising pneumatic pulsing valves for each gas flow (to control the composition/blending); 1 s cycle with a minimum pulse time of 20 ms (for controlling the duration and interval of the gas pulsing); a mass flow sensor downstream of the pulsing valves allowing a flow range of from 0.0013 mL/min to 550 mL/min.

Geometries of different reactor sizes are depicted in the following Table (reproduced from Bareither, R., et al., Biotechnol. Bioeng. 110 (2013) 3126-3138; Table II).

| **cell culture characteristic** | **cell culture volume of 250 mL** | **cell culture volume of 3 L** | **cell culture volume of 500 L** |
|---|---|---|---|
| working volume [L] | 0.10-0.25 | 1.5-2.5 | 200-500 |
| *HL*/*D*t | 1.3 | 1.2 | 1.21 |
| impeller type | pitched blade | pitched blade/marine | marine |
| number of impellers | 2 | 2 | 2 |
| *D*1/*D*T | 0.43 | 0.38 | 0.36 |
| *C*/*D*T | 0.36 | 0.23 | 0.2 |
| geometric similarity | 0.97 | 1.00 | 0.9 |
| Vsg (ms⁻¹) | 0.0031 | 0.003 | 0.004 |
| agitation (rpm) | 200-800 (360) | 50-400 (200) | 50-350 (70) |
| tip speed (ms⁻¹) | 0.27-1.02 (0.52) | 0.12-1.02 (0.69) | 0.97-6.78 (1.16) |
| Pg/VL (kW/m³) | 0.01-0.445 (0.035) | 0.002-0.5 (0.035) | 0.14-0.45 (0.036) |
| kLa (h-¹) | 2.5-8.5 (10) | 4-15 (12) | (8.74) |

| **cell culture characteristic** | **cell culture volume of 5,000 L** | **cell culture volume of 18,000 L** |
|---|---|---|
| working volume [L] | 2,000-5,000 | 8,000-18,000 |
| *H*_{L}/*D*ₜ | 1.65 | 1.66 |
| impeller type | A320 | A320 |
| number of impellers | 3 | 3 |
| *D*ₗ/*D*_{T} | 0.5 | 0.49 |
| *C*/*D*T | 0.27 | 0.29 |
| geometric similarity | 0.923 | 0.942 |
| Vsg (ms⁻¹) | 0.0009 | 0.0013 |
| agitation (rpm) | 10-100 (40) | 10-70 (31) |
| tip speed (ms⁻¹) | 0.41-4.13 (1.53) | 0.64-4.47 (1.6) |
| *P*g/VL (kW/m³) | 0.001-0.445 (0.03) | 0.01-0.397 (0.034) |
| k_{L}a (h⁻¹) | 5-9 (9.4) | 4.13 (15) |

A suitable SUSVB shall have at least equal H_{L}/Dᵢ- and D_{T}/Dᵢ-ratios, impeller spacing, as well as baffles as intended to be used for the large-scale fermenter. The power input should be in the rage of 0.01 kW/m³ to 0.4 kW/m³ and the k_{L}a value should be in the range of 1 1/h to 15 1/h. Dissolved oxygen should be controllable to 20 % air saturation or more, whereas the dissolved carbon dioxide (CO₂) content should be in the range of 35 mmHg to 80 mmHg. Temperature should be controlled in the range of from 32 °C to 38 °C and the pH value in the range of from pH 6.8 to pH 7.2. The feeding should be in the range of 20 pg/cell/day to 90 pg/cell/day and automation for feed control and different feeding strategies (linear ramp, exponential, constant, bolus additions) should be provided. The working volume to enable parallel processing and sampling should be in the range of 20 ml to 300 ml, preferably in the range of 60 ml to 255 mL for development and product quality analysis. The SUSVB should allow for nutrient addition triggered by sensors or feeding via pH stat or dO stat.

Generally, for the fed batch cultivation of a CHO cell line, e.g. a CHO K1 cell line, expressing a monoclonal antibody any medium, such as commercial CD-CHO medium or any other serum-free medium can be used. Inoculation is performed using standard conditions, such as e.g. an inoculation cell density of 2×10⁵ viable cells/mL. The SUSVBs can be inoculated with a shaker flask seed culture (e.g. from a humidified incubator, 36.5 °C, 5 % CO₂; expanded every 3 to 4 days within growth phase and above 1×10⁶ viable cells/mL). The pH should be controlled at about pH 6.9 to 7.1 (1 molar sodium bicarbonate solution as base and CO₂ as acid), the temperature should be controlled to be about 36.5 °C, dO2 should be controlled to be at about 30% air saturation, agitation should be controlled at a specific power input, and a minimum air sparging rate should be set for stripping of CO₂, such as 0.0125 vvm. A multiple nutrient feed solution can be added based on sensor readout or based on a fixed feeding scheme, e.g. on days 4, 6, 8, and 11. The feed solution should not exceed a predefined volume, such as, e.g., 5% of the working volume. Glucose limitation should be avoided, e.g. by addition of a concentrated glucose solution, e.g. 40 mmol/L. Antifoam can be added if required. For analysis samples are removed at fixed time points, e.g. daily, to determine cell viability, glucose, lactate, osmolarity, pH, whereas dissolved gases (dO2, dCO2) are determined offline, e.g. with a blood gas analyzer.

In general, the SUSVBs according to the current invention can be used
- for determining fermentation conditions;
- for determining bioprocess parameters.

In general, in one preferred embodiment, such single-use small volume bioreactors (SUSVBs) are at least partially made from non-metal, non-glass, polymeric materials. As disclosed in US 9,938,493 (incorporated herein in its entirety by reference) known cell culture containers are constructed from multiple layers, wherein the inner layer, i.e. the layer which gets into contact with the cell cultures, is made of a polymer material. Usually at least this layer consists of polyethylene (PE) or ethyl-vinyl acetate (EVA).

As used herein, the terms "single-use cell culture container" and "single-use small volume bioreactor (SUSVB)", which can be used interchangeably, denote a cell cultivation vessel with a working volume of 300 mL or less, made of a single- or multi-layer polymer material for cultivating mammalian or bacterial cells for the production of biological materials. The cell culture container or small volume bioreactor for use in accordance with the invention can be of any shape. The term "single-use" as used herein denotes that the cell culture container or small volume bioreactor is used only once for the cultivation of cells. This does not impart the fact that the cell culture container or small volume bioreactor is sterilized more than once prior to the use. Thus, before filling the cell culture container or small volume bioreactor with cultivation medium or cells it is treated with microorganism killing radiation, such as beta- or gamma-radiation. This needs to be done to kill all microorganisms present inside the device, which can disturb and affect growth of the cultivated cells.

In one embodiment, the occurrence of an extended lag phase in the cultivation of a mammalian cell in a single-use cell culture container or SUSVB according to the invention using a serum-free medium is prevented by the treatment of the container or SUSVB with inert gas prior to the application of sterilizing radiation. In one embodiment, the single-use cell culture container or SUSVB is at least partially made of polymer material. In one embodiment, the sterilizing radiation is beta irradiation or gamma radiation.

### EXPERIMENTAL RESULTS

In the following experimental results obtained with an exemplary SUSVB according to the invention are outlined. These are presented as mere exemplification and shall not be construed as limitation. The true scope of the invention is set forth in the appended claims.

The SUSVB according to the current invention comprising an in-situ glucose sensor has been compared to a standard SUSVB without an additional glucose sensor, i.e. with a single in-situ sensor. The respective experiments are summarized in the following Table.

| **run** | **clone** | **Condition** | **in situ glucose sensor** |
|---|---|---|---|
| RE01 | P199 | standard | none |
| RE02 | P199 | 1 g/L glucose | low glucose sensor |
| RE04 | P199 | 5 g/L glucose | high glucose sensor |
| RE05 | P438 | standard | none |
| RE06 | P438 | 1 g/L glucose | low glucose sensor |
| RE07 | P438 | 3 g/L glucose | low glucose sensor |
| RE08 | P438 | 5 g/L glucose | high glucose sensor |
| RE09 | P465 | standard | none |
| RE10 | P465 | 1 g/L glucose | low glucose sensor |
| RE12 | P465 | 5 g/L glucose | high glucose sensor |

RE01/RE05/RE09 were each a 10-day cultivation with off-line glucose analysis using a small volume bioreactor, i.e. with only one in-situ sensor sterilized once with beta-radiation. These are comparative experiments.

RE02/RE04/RE06/RE07/RE08/RE10/RE12 were each a 16-day cultivation using a SUSVB according to the current invention, i.e. with two in-situ sensors sterilized twice with beta- and gamma-radiation.

Two different in-situ glucose sensors were used in the SUSVB according to the invention: a high glucose and a low glucose sensor. The difference of both sensor types is a different type of membrane layer over the sensor. This difference results in different diffusions time of glucose to the enzyme in the sensor.

RE02/06/07/10 were each with a low glucose sensor with a measurement range of 0-3 g/L. RE04/08/12 were each with a high glucose sensor with a measurement range of 0-8 g/L.

Feed 1 (contains glucose) was stopped at day 9 in vessels RE02/04/06/07/08/10/12 to bring glucose level down to the measurement range of the glucose sensors to start glucose control via the sensor.

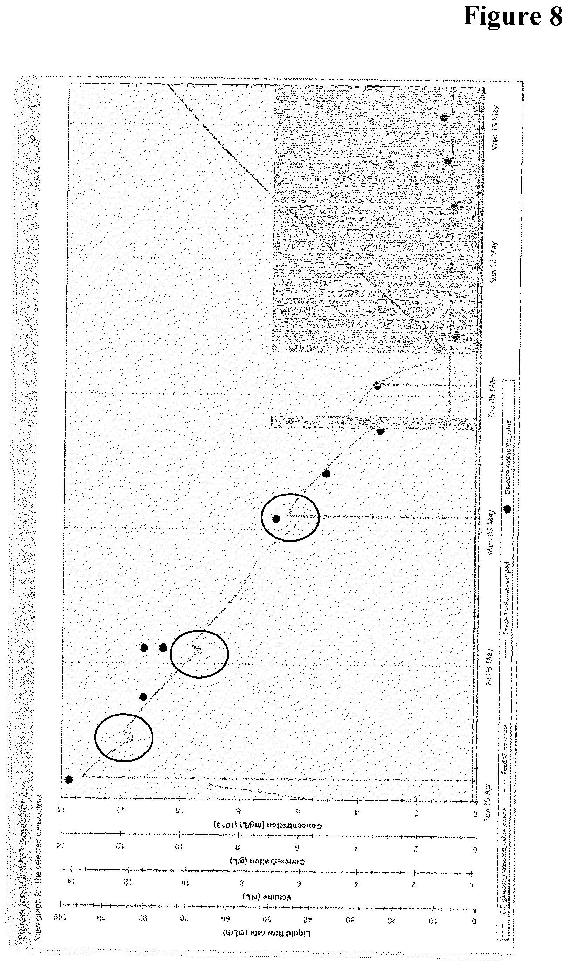

An exemplary glucose curve obtained for RE02 is shown in Figure 8. The green curve is the glucose concentration as determined with the in-situ sensor. Each steep step of the green curve (Tue 30 Apr; Mon 06 May; Thu 09 May) is a recalibration step of the sensor. It can be seen that the glucose sensor shows low drift. No recalibration for 10 days was necessary. The red line is the cumulative volume/amount of fed glucose solution. The pink lines represent the action times/flow-rates of the glucose solution feeding pump. At the encircled time points a bolus feed without glucose was given. At day 9 the feeding of feed 1 that also contained glucose was stopped. The black dots represent the control glucose values obtained by sampling and Cedex BioHT offline analysis.

One aspect as reported herein is a SUSVB for the culturing of animal cells. In one embodiment the SUSVB comprises
a) a single use small volume cultivation vessel according to the invention which is suitable for receiving a cultivation medium and animal cells to be cultured therein,
b) a stirrer system,
c) a glucose sensor,
d) a gas inlet at the bottom of the cultivation vessel, and
e) at least one inlet for adding correcting and/or feeding solutions.

One aspect as reported herein is a method for producing a polypeptide, especially an antibody, comprising the following steps:
a) culturing a cell comprising a nucleic acid encoding the polypeptide in a SUSVB according to the invention,
b) recovering the polypeptide from the cultivation medium or the cells, and
c) optionally purifying the polypeptide and thereby producing the polypeptide.

One aspect as reported herein is a method for culturing animal or bacterial cells, characterized in that the animal or bacterial cells are cultured in a SUSVB as reported herein, optionally thereby a product is produced.

One aspect as reported herein is the use of the SUSVB for the production of polypeptides or antibodies or viruses.

In one embodiment the ratio of the diameter of the impeller d to the diameter of the SUSVB D when the stirrer system is placed in the cultivation vessel is in the range between 0.2 and 0.8, in another embodiment in the range between 0.3 and 0.6, in a further embodiment in the range between 0.31 and 0.39, or in also an embodiment about 0.34. In a further embodiment the pitch of the stirrer blades of the axially-conveying impeller is between 10° and 80°, in another embodiment between 24° and 60°, or in a further embodiment between 40° and 50° relative to the shaft axis. In one embodiment all impeller have a ratio of the diameter of the conveying element d to the diameter of the cultivation vessel D of from 0.32 to 0.35.

In one embodiment, the purifying is a multistage chromatographic process. In another embodiment, the purifying comprises an affinity chromatography, a cation exchange chromatography and an anion exchange chromatography.

In one embodiment, the culturing is a semi-continuous culturing.

In one embodiment, the polypeptide is an antibody or an antibody derivative.

In one embodiment, the cultivation medium is an aqueous medium, which is suitable for the cultivation of prokaryotic and eukaryotic cells. In another embodiment, the cultivation medium is a Newtonian liquid. In one embodiment, the stirrer system is operated at a power input of from 0.01 W/kg to 1 W/kg. In a further embodiment, the stirrer system is operated at a power input of from 0.04 W/kg to 0.5 W/kg. In still another embodiment, the flow induced by the stirrer system in the cultivation medium is a turbulent flow. In another embodiment the cultivation medium has a viscosity of 3 mPas*s or less. In another embodiment the viscosity is 2 mPas*s or less.

In one embodiment, the SUSVB is a submersed gassed stirred tank reactor.

In another embodiment, the animal cell is a mammalian cell. In yet a further embodiment the cell is a CHO cell, a BHK cell, an NS0 cell, a COS cell, a PER.C6 cell, a Sp2/0 cell, an HEK 293 cell or a hybridoma cell.

In order to achieve high product titer and a good product quality the operating mode of the SUSVB according to the invention has an important role in addition, e.g., to the cell line development, the media composition and the dimensioning of the SUSVB.

A distinction can be made between the operating modes batch or batch processes, fed batch or feeding processes, continuous processes with or without cell retention (for example perfusion or chemostat) as well as semi-continuous processes such as e.g. internal or external dialysis.

The SUSVB has an upper portion, a middle portion and a lower portion, wherein the longitudinal axis of the SUSVB extends from the middle or center of the upper portion to the middle or center of the lower portion.

The SUSVB has a substantially circular cross-section when viewed perpendicular to the longitudinal axis. The diameter can be the same in the upper and the lower section or the lower section can have a smaller diameter than the upper section.

The upper portion of the SUSVB may further comprise a gas discharge outlet means, and/or one or more inlet means.

The lower portion of the SUSVB may further comprise one or more liquid media inlet means, and/or a gas inlet means.

At least the lower or the middle portion of the SUSVB may further comprise a heat exchange jacket mounted to the outside wall of the cultivation vessel.

The conveying-elements of the stirrer system are set into rotation by a shaft, which is coupled to a suitable mechanism for inducing a rotation thereof. The shaft extends along the longitudinal axis of the SUSVB and, thus, the shaft has a vertically oriented axis of rotation. The shaft does not extend to the bottom of the SUSVB but to a point well above the bottom of the SUSVB and also well above an optional gas-sparger at the bottom of the SUSVB. The shaft is operably coupled to a drive shaft by a suitable coupling mechanism. Beside a means for coupling the shaft to the drive shaft the shaft may in addition compromises further means for individually coupling the impellers to the shaft. The impellers of the stirrer system are normally coupled to the shaft at a position that is/will be below the surface of the cultivation medium in the SUSVB once the stirrer system is submersed in the cultivation medium. The surface is determined when the cultivation medium is static, i.e. not being circulated.

The SUSVB according to the invention is a baffled SUSVB. In another embodiment, the SUSVB according to the invention comprises two or four baffles. A "baffle" denotes a plate placed inside a cultivation vessel in the same direction as the shaft axis and extending radially into the cultivation vessel towards the stirrer. The baffle is generally rectangular in shape. In one embodiment, not according to the invention, the baffle is placed at a distance b_{d} to the inner wall of the SUSVB. In another embodiment, the baffles are spaced at equal distance to each other around the circumference of the inside of the SUSVB.

The components of the SUSVB are dimensioned in a way that they can exert their intended function, i.e. the SUSVB can take up the cultivation medium, and the stirrer system can mix the medium and disperse added compound. Thus, the stirrer system has a diameter that allows for an unhampered rotation within the SUSVB.

With the SUSVB as reported herein a high cell density cultivation as, e.g., perfusion cultivation can be carried out.

The culturing is carried out in one embodiment at a rotation speed of the stirrer system at which a Reynolds-number independent constant power input to the cultivation medium can be achieved, i.e. during the culturing a turbulent cultivation medium flow in the cultivation vessel is provided. It is possible with a SUSVB according to the invention to cultivate shear sensitive mammalian cells at a low rotation speed of the stirrer system.

The form of the cultivation vessel of the SUSVB is not limited. In one embodiment, the cultivation vessel is a cylindrical vessel. In another embodiment, the cultivation vessel is a stirred tank reactor-like vessel. The cultivation vessel may have any dimension. In one embodiment, the cultivation vessel has a working volume of from 20 ml to 350 ml, in one preferred embodiment of 55 ml to 265 ml.

In general, submerse gassed cultivation vessels are used in cell culture. In these cases, a one-stage or two-stage or three-stage axially-conveying stirrer system is mainly used. In case a one-stage or two-stage axially-conveying stirrer system is used this generates a flow profile which is essentially parallel to the rotary shaft of the employed stirrer.

In one embodiment, the stirrer comprises 1 to 5 axially-conveying elements, or in another embodiment 1 to 3 axially-conveying elements, or in also an embodiment 1 or 2 axially-conveying elements or even only a single axially-conveying element. In one embodiment, one axially-conveying element is situated in the upper four fifths of the stirrer shaft determined from the head of the stirrer and is at a maximum distance of h_{4/5} to the head of the stirrer. In also an embodiment one axially-conveying element is located at a maximum distance of 0.8 h and/or one axially-conveying element is located at a maximum distance of 0.2 h. In a further embodiment, the axially-conveying elements form together a single element. In one embodiment, the diameter of all axially-conveying elements is identical. In another embodiment the axially-conveying elements is selected independently of each other from a propeller agitator, pitched-blade agitator, or inclined-blade agitator.

In a further embodiment, all conveying elements rotate with the same number of rotations per time unit around the shaft axis of the stirrer when the stirrer is operated in a SUSVB. In one embodiment, the conveying elements are permanently joined together and the stirrer consists of one part, i.e. all elements are driven by the same rotary shaft and have the same number of rotations per time unit around the shaft axis of the stirrer.

The ratio d/D of stirrer diameter (d) to cultivation vessel diameter (D) is in one embodiment of from 0.2 to 0.8, in another embodiment of from 0.3 to 0.6, and in a further embodiment of from 0.33 to 0.5. In another embodiment, the ratio h/d of blade height (h) to stirrer diameter (d) is of from 0.5 to 5, in another embodiment of from 1 to 4, and in a further embodiment of from 1 to 3. In yet another embodiment the ratio b/d of impeller blade width (b) to stirrer diameter (d) is of from 0.05 to 0.3, in another embodiment of form 0.1 to 0.25.

The term "of from ... to" denotes a range including the listed boundary values.

In one embodiment the stirrer diameter (d) is selected from 5 mm, 6 mm, 7 mm, 8 mm, 10 mm, 12 mm, 14 mm, 16 mm, 18 mm and 20 mm.

In one embodiment the impeller blade width (b) is selected from, 0.42 mm, 0.60 mm, 0.89 mm, 1.08 mm, 1.33 mm.

In one embodiment, the one radially-conveying element is an anchor impeller.

The term "approximately" denotes that the given value is the center point of a range spanning plus/minus 10 % around the value. If this value is a percentage value then "approximately" denotes also means plus/minus 10 %, but the value 100 % cannot be exceeded.

In one embodiment, the axially-conveying element is an inclined-blade impeller.

The ratio h_{SB}/b of the height of the axially-conveying element and/or the width of the blades of the radially-conveying element is of from 0.5 to 4, in another embodiment of from 0.8 to 3, and in a further embodiment of from 1 to 2. In another embodiment the pitch of the stirrer blades of the inclined-blade impeller is of from 10° to 80°, in a further embodiment of from 24° to 60°, and in also an embodiment of from 40° to 50° relative to the shaft axis of the stirrer.

In one embodiment, the radially-conveying element has of from 1 to 8 blades, in another embodiment of from 1 to 4 blades, and in a further embodiment 4 blades. The axially-conveying element has in one embodiment of from 1 to 10 blades, in another embodiment of from 2 to 6 blades, and in a further embodiment 4 blades. In another embodiment, the radially-conveying and axially-conveying elements have the same number of blades.

In one embodiment, the stirrer has a height of from 20 mm to 500 mm.

In one embodiment, the stirrer and the SUSVB form a functional unit, i.e. the stirrer is within the cultivation vessel of the SUSVB and can rotate within the cultivation vessel without any spatial limitation.

The SUSVB according to the invention is a stirred tank reactor-like SUSVB.

The cultivation vessel is an aerated or submerse gassed stirred reactor-like vessel.

The cultivation vessel comprises 2 (114, 126) or 4 baffles (114, 126, 125, 128). In another embodiment the baffles are spaced at equal distance to each other around the circumference of the inside surface of the cultivation vessel. The ratio dID of stirrer diameter (d) to cultivation vessel diameter (D) is in one embodiment of from 0.2 to 0.8, in another embodiment of from 0.3 to 0.6, and in a further embodiment of from 0.33 to 0.5. In another embodiment, the ratio H/D of filling height of the cultivation vessel (H) to cultivation vessel diameter (D) is of from 1.0 to 2.5, in a further embodiment of from 1.1 to 20, and in a further embodiment of from 1.4 to 1.8. In one embodiment, the cultivation vessel has a working volume of from 20 ml to 350 ml.

In one embodiment the ratio of the height difference (Δh) of two axially-conveying elements to the cultivation vessel diameter (D) is at least 0.75.

The following examples, sequences and figures are provided to aid the understanding of the present invention, the true scope of which is set forth in the appended claims. It is understood that modifications can be made in the procedures set forth without departing from the spirit of the invention.

### Abbreviations:

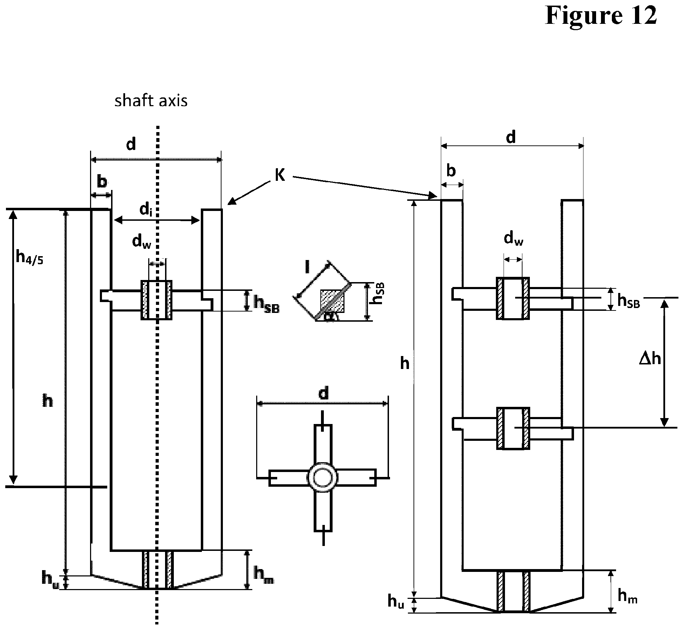

The abbreviations used in this application have the following meanings (see also Figure 12):
- b:: width of the blades of the radially-conveying element
- d:: agitator total outer diameter
- d_{w}:: diameter of the shaft
- h:: height of the stirrer blades of the radially-conveying element
- hₘ:: height of the fastening sleeve
- h_{SB}:: height of an axially-conveying element
- hᵤ:: height of the reducer
- Δh:: height difference of two axially-conveying elements
- l:: length of the stirrer blades of an axially-conveying element
- α:: blade pitch of the blades of an axially-conveying element
- z:: number of stirrer blades per stirrer
- dᵢ:: inner distance between the stirrer blades of the radially-conveying element
- h_{4/5}:: 4/5 height from above of h
- K:: stirrer head, i.e. the highest point of the stirrer when it is not attached to a rotary shaft
- D:: cultivation vessel inner diameter
- H:: filling height of the cultivation vessel.

### Descrintion of the Figures

- **Figure 1:**: Side view of an exemplary single use small volume bioreactor (SUSVB) according to the current invention containing a glucose sensor (111).
- **Figure 2:**: Side view of an exemplary SUSVB according to the invention with dimension and volume annotations.
- **Figure 3:**: Top view of an exemplary SUSVB according to the invention showing the glucose sensor (111) fitted to the supply port area (133).
- **Figure 4:**: Top-down view of an exemplary SUSVB according to the current invention containing a glucose sensor (111).
- **Figure 5:**: Enlarged view of the part of the SUSVB according to the invention comprising the glucose sensor (111).
- **Figure 6:**: Side view of the lower part of an exemplary SUSVB according to the invention comprising a glucose sensor (111) embedded in the cultivation medium (129).
- **Figure 7:**: Schematic view of an exemplary glucose sensor (111).
- **Figure 8:**: Temporal plot of on-line determined glucose concentration (green line), off-line determined glucose concentrations (black filled circles); added glucose solution volume (red line) and glucose pump action (purple line) of the cultivation RE02.
- **Figure 9:**: Temporal plot of on-line determined glucose concentration (green line), off-line determined glucose concentrations (black filled circles); added glucose solution volume (red line) and glucose pump action (purple line) of the cultivation RE06.
- **Figure 10:**: Temporal plot of on-line determined glucose concentration (green line), off-line determined glucose concentrations (black filled circles); added glucose solution volume (red line) and glucose pump action (purple line) of the cultivation RE10.

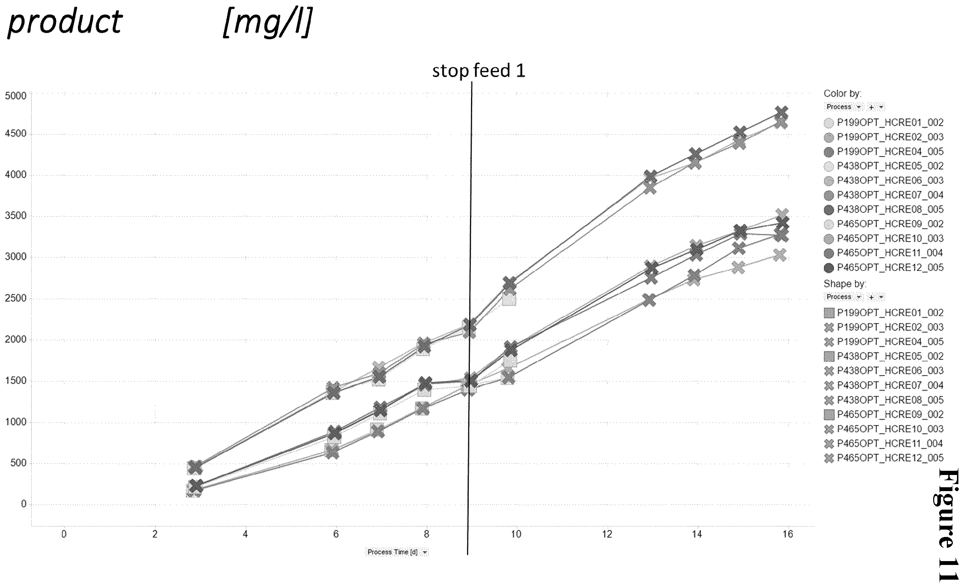

- **Figure 11:**: Temporal plot of antibody concentration of the fermentations RE01-RE02, and RE04-RE12.
- **Figure 12:**: Schematic diagram of various embodiments of the combination stirrer according to the invention; b: width of the stirrer blade; d: stirrer diameter; d_{w}: diameter of the rotary shaft; h: height of the stirrer blade of the radially-conveying stirrer; hₘ: height of the fastening sleeve; hss: height of the axially-conveying stirrer; hᵤ: height of the reducer; l: length of the stirrer blade of the axially-conveying stirrer; α: blade pitch of the axially-conveying stirrer; z: number of stirrer blades per stirrer; dᵢ: inner distance between the stirrer blades of the radially-conveying stirrer; h_{4/5}: 4/5 height from above of h; K: stirrer head.

### Example 1

### Cultivation conditions for RE01-RE12

The cultivations were performed with a starting cell density of about 1.5 * 10E7 cells/ml in a total volume of 170 ml. The cultivation medium was a serum-free chemically defined medium. The cultivation temperature was set to 35 °C, the gassing rate was set to 5-5.5 ml/min, the agitation rate was set to 450-500 rpm, and the pH was set to pH 7. PH-control was performed by adding a 1 M sodium carbonate solution or CO₂ on top of the gassing rate. Defoamer was added at the beginning and during the cultivation when needed. Feed 1 containing glucose was added continuously with a pre-defined rate until stopped. Feed 2 not containing glucose was added as bolus-feed on days 1, 3 and 6.

In cultivations with an in-situ glucose sensor according to the invention sensor-dependent glucose feeding was started (feed 3) after the end of feed 1 once the determined in-situ glucose concentration dropped below a threshold value. The threshold value was lowered during the process.

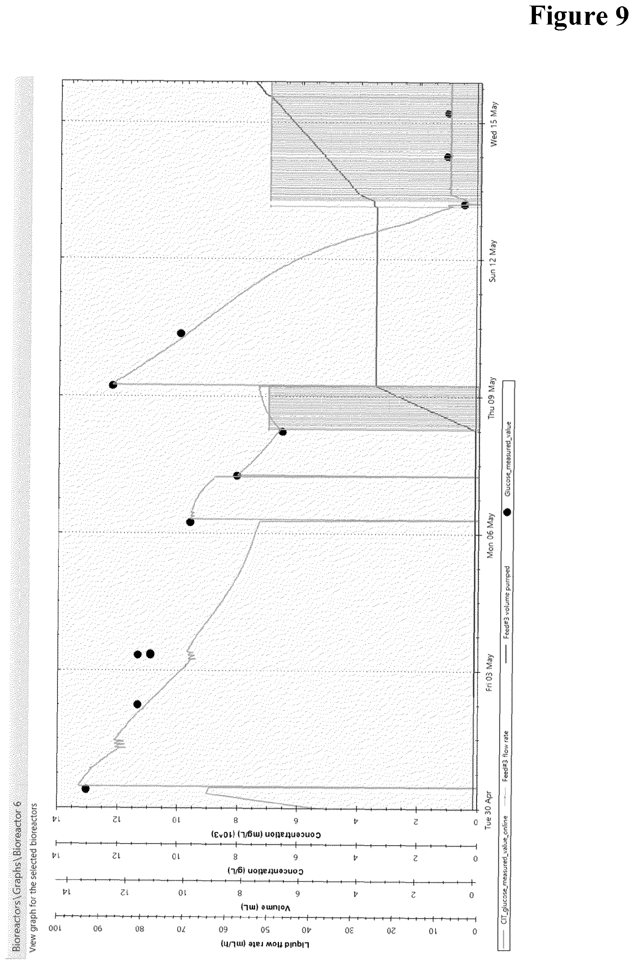

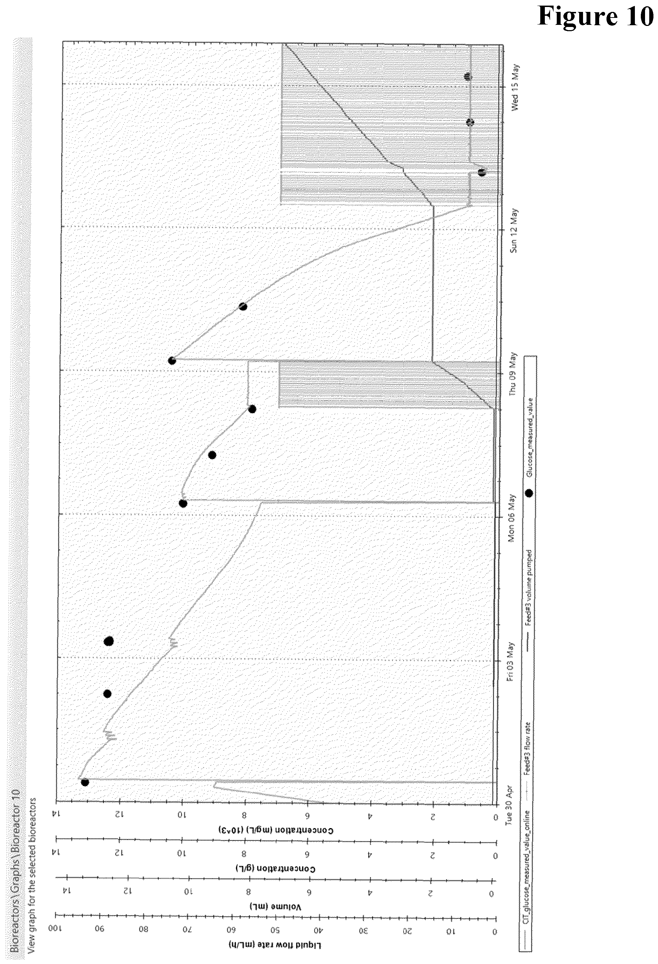

Figures 8 to 10 show plots of exemplary cultivation and Figure 12 depicts the product concentration profile.

## Claims

1. A small volume bioreactor comprising
a cultivation vessel (105) that
- has a working volume of from 20 ml to 350 ml,
- comprises a stirrer shaft (108) with at least one impeller (112) affixed thereto,
- comprises a feed pipe (107) comprising i) a sparging tube connected to a sparger (127) at its end, and ii) at least one feed line (118) with an opening at its end,
- comprises two or more baffles (114; 126) extending from the wall of the cultivation vessel (105) perpendicular in the direction to the center of the cultivation vessel (105), and
- a reactor head plate (104),
wherein the reactor head plate (104) comprises
- a fitting (122) for connecting the drive axis of a motor to the stirrer shaft (108),
- a sparger gas inlet (116) connected to the sparging tube in the feed pipe (107), optionally a gas inlet connected to the headspace (132),
- a gas outlet connected to the headspace of the cultivation vessel (117),
- at least one inlet for liquids of a feed line (118) being part of the feed pipe (107),
- one in-situ sensor port (130) with a pH electrode (101) mounted thereto, and
- a supply port area (133) comprising the sparger gas inlet and the inlet of the at least one feed line (118),
wherein the cultivation vessel (105) and the reactor head plate (104) are both substantially made of non-metal material,
whereby the small volume bioreactor comprises an in-situ glucose sensor.

2. The small volume bioreactor according to claim 1, wherein the small volume bioreactor is a single-use small volume bioreactor.

3. The small volume bioreactor according to any one of claims 1 to 2, wherein the glucose sensor is a screen-printed electrode coated with an immobilized enzyme.

4. The small volume bioreactor according to any one of claims 1 to 3, wherein the glucose sensor base material is polymer USP class VI.

5. The small volume bioreactor according to any one of claims 1 to 4, wherein the glucose sensor determines the glucose concentration every 20 seconds and/or determines the change of the glucose concentration.

6. The small volume bioreactor according to any one of claims 1 to 5, wherein the determined glucose concentration value is transmitted wireless or by cable from the glucose sensor to a computer.

7. The small volume bioreactor according to any one of claims 1 to 6, wherein the reactor head plate (104) further comprises a sampling port (102).

8. The small volume bioreactor according to any one of claims 1 to 7, wherein the in-situ glucose sensor passes the head plate (104) in or at the supply port area (133).

9. A method for cultivating a mammalian cell using a small volume bioreactor according to any one of claims 1 to 8.

## Patentansprüche

1. Kleinvolumenbioreaktor, umfassend
ein Kultivierungsgefäß (105), das
- ein Arbeitsvolumen von 20 ml bis 350 ml hat,
- eine Rührstange (108) mit mindestens einem daran befestigten Flügelrad (112) umfasst,
- ein Zulaufrohr (107), umfassend i) einen Perlgasschlauch, der mit einer Perlgasvorrichtung (127) an seinem Ende verbunden ist, und ii) mindestens eine Zulaufleitung (118) mit einer Öffnung an ihrem Ende, umfasst,
- zwei oder mehr Prallbleche (114; 126) umfasst, die sich von der Wand des Kultivierungsgefäßes (105) senkrecht in der Richtung zur Mitte des Kultivierungsgefäßes (105) erstrecken, und
- eine Reaktorkopfplatte (104) umfasst,
wobei die Reaktorkopfplatte (104) Folgendes umfasst
- ein Anschlussstück (122) zum Verbinden der Antriebsachse eines Motors mit der Rührstange (108),
- einen Perlgaseinlass (116), der mit dem Perlgasschlauch in dem Zulaufrohr (107) verbunden ist, gegebenenfalls einen Gaseinlass, der mit dem Kopfraum (132) verbunden ist,
- einen Gasauslass, der mit dem Kopfraum des Kultivierungsgefäßes (117) verbunden ist,
- mindestens einen Einlass für Flüssigkeiten einer Zulaufleitung (118), die Teil des Zulaufrohres (107) ist,
- eine In-situ-Sensoranschlussstelle (130) mit einer daran montierten pH-Elektrode (101) und
- einen Versorgungsanschlussstellenbereich (133), der den Perlgaseinlass und den Einlass der mindestens einen Zulaufleitung (118) umfasst,
wobei das Kultivierungsgefäß (105) und die Reaktorkopfplatte (104) beide im Wesentlichen aus einem Nichtmetallmaterial gefertigt sind,
wobei der Kleinvolumenbioreaktor einen In-situ-Glukosesensor umfasst.

2. Kleinvolumenbioreaktor nach Anspruch 1, wobei der Kleinvolumenbioreaktor ein Einweg-Kleinvolumenbioreaktor ist.

3. Kleinvolumenbioreaktor nach einem der Ansprüche 1 bis 2, wobei der Glukosesensor eine Siebdruckelektrode ist, die mit einem immobilisierten Enzym beschichtet ist.

4. Kleinvolumenbioreaktor nach einem der Ansprüche 1 bis 3, wobei das Glukosesensor-Grundmaterial Polymer USP Klasse VI ist.

5. Kleinvolumenbioreaktor nach einem der Ansprüche 1 bis 4, wobei der Glukosesensor die Glukosekonzentration alle 20 Sekunden bestimmt und/oder die Veränderung der Glukosekonzentration bestimmt.

6. Kleinvolumenbioreaktor nach einem der Ansprüche 1 bis 5, wobei der bestimmte Glukosekonzentrationswert drahtlos oder über ein Kabel von dem Glukosesensor an einen Computer gesendet wird.

7. Kleinvolumenbioreaktor nach einem der Ansprüche 1 bis 6, wobei die Reaktorkopfplatte (104) femer eine Probennahmeanschlussstelle (102) umfasst.

8. Kleinvolumenbioreaktor nach einem der Ansprüche 1 bis 7, wobei der In-situ-Glukosesensor die Kopfplatte (104) im oder am Versorgungsanschlussstellenbereich (133) passiert.

9. Verfahren zum Kultivieren einer Säugerzelle unter Verwendung eines Kleinvolumenbioreaktors nach einem der Ansprüche 1 bis 8.

## Revendications

1. Bioréacteur de petit volume comprenant
une cuve de culture (105) qui
- a un volume de travail de 20 ml à 350 ml,
- comprend une tige d'agitateur (108) avec au moins une pale (112) fixée à celle-ci,
- comprend un conduit d'alimentation (107) comprenant i) un tube de barbotage relié à un barboteur (127) au niveau de son extrémité, et ii) au moins une ligne d'alimentation (118) avec une ouverture au niveau de son extrémité,
- comprend deux déflecteurs (114 ; 126) ou plus s'étendant depuis la paroi de la cuve de culture (105) perpendiculaire en direction du centre de la cuve de culture (105), et
- une plaque de tête de réacteur (104),
dans lequel la plaque de tête de réacteur (104) comprend
- un raccord (122) pour relier l'axe d'entraînement d'un moteur à la tige d'agitateur (108),
- une entrée de gaz de barboteur (116) reliée au tube de barbotage dans le conduit d'alimentation (107), éventuellement une entrée de gaz reliée à l'espace de tête (132),
- une sortie de gaz reliée à l'espace de tête de la cuve de culture (117),
- au moins une entrée pour les liquides d'une ligne d'alimentation (118) faisant partie du conduit d'alimentation (107),
- un orifice de capteur in situ (130) avec une électrode de pH (101) montée sur celui-ci, et
- une zone d'orifice d'alimentation (133) comprenant l'entrée de gaz de barboteur et l'entrée de l'au moins une ligne d'alimentation (118),
dans lequel la cuve de culture (105) et la plaque de tête de réacteur (104) sont toutes deux essentiellement fabriquées en matériau non métallique,
moyennant quoi le bioréacteur de petit volume comprend un capteur de glucose in situ.

2. Bioréacteur de petit volume selon la revendication 1, dans lequel le bioréacteur de petit volume est un bioréacteur de petit volume à usage unique.

3. Bioréacteur de petit volume selon l'une quelconque des revendications 1 à 2, dans lequel le capteur de glucose est une électrode sérigraphiée revêtue d'une enzyme immobilisée.

4. Bioréacteur de petit volume selon l'une quelconque des revendications 1 à 3, dans lequel le matériau de base du capteur de glucose est un polymère USP de classe VI.

5. Bioréacteur de petit volume selon l'une quelconque des revendications 1 à 4, dans lequel le capteur de glucose détermine la concentration en glucose toutes les 20 secondes et/ou détermine le changement de la concentration en glucose.

6. Bioréacteur de petit volume selon l'une quelconque des revendications 1 à 5, dans lequel la valeur de la concentration en glucose déterminée est transmise sans fil ou par câble depuis le capteur de glucose jusqu'à un ordinateur.

7. Bioréacteur de petit volume selon l'une quelconque des revendications 1 à 6, dans lequel la plaque de tête de réacteur (104) comprend en outre un orifice d'échantillonnage (102).

8. Bioréacteur de petit volume selon l'une quelconque des revendications 1 à 7, dans lequel le capteur de glucose in situ traverse la plaque de tête (104) dans ou au niveau de la zone d'orifice d'alimentation (133).

9. Procédé de culture d'une cellule de mammifère en utilisant un bioréacteur de petit volume selon l'une quelconque des revendications 1 à 8.
